# EUROPÄISCHE PATENTANMELDUNG

(11) **EP 4 278 947 A1**
(43) Veröffentlichungstag der Anmeldung: **22.11.2023**
(21) Anmeldenummer: 23172724.9
(22) Anmeldetag: 11.05.2023
(51) Int. Cl.: A61B 1/00, A61B 1/015, A61B 1/12

(54) **WIEDERVERWENDBARER SPÜLSCHAFT FÜR EIN ENDOSKOP**

(30) Priorität: 19.05.2022 DE 102022112589
(71) Anmelder: Henke-Sass, Wolf GmbH, 78532 Tuttlingen (DE)
(72) Erfinder: DOMINKE, Ina, 78073 Bad Dürrheim (DE); SEEH, Daniel, 78194 Immendingen (DE)
(74) Vertreter: Patentanwälte Geyer, Fehners & Partner mbB

(57) **Zusammenfassung**

Es wird ein wiederverwendbarer Spülschaft (1) für ein Endoskop (15) bereitgestellt,
wobei der Spülschaft (1)
einen Endabschnitt (2),
ein mit dem Endabschnitt (2) verbundenes Hüllrohr (5),
eine am Endabschnitt (2) ausgebildete Fixiereinheit (11) und
einen Spülanschluss (7), der in Fluidverbindung mit dem Hüllrohr (5) steht, aufweist,
wobei der Spülschaft (1) einen sich durch den Endabschnitt(2) und das Hüllrohr (5) erstreckenden Endoskopkanal (6) aufweist, so dass der Endoskopschaft (17) mit seinem distalen Ende (20) in den Endoskopkanal (6) einführbar ist, bis für das Endoskop (15) eine Arbeitsposition erreicht ist,
wobei das Hüllrohr (5) an seinem vom Endabschnitt (2) wegweisenden distalen Ende (25) einen axialen Anschlag (26), der die maximale Einführtiefe des Endoskopschafts (17) in den Endoskopkanal begrenzt, sowie mindestens zwei radiale Anschläge (27, 28), die die radiale Position des Endoskopschafts im Hüllrohr vorgeben, aufweist,
wobei der Spülschaft (1), wenn der Endoskopschaft in den Endoskopkanal eingeführt und die Arbeitsposition erreicht ist, mittels der Fixiereinheit (11) so am Endoskop (15) fixiert ist, dass das distale Ende (20) des Endoskopschafts am axialen Anschlag anliegt und die radiale Position des Endoskopschafts im Hüllrohr durch die mindestens zwei radialen Anschläge so vorgegeben ist, dass sich ein Spülkanal (30) innerhalb des Hüllrohrs (5), der in Fluidverbindung mit dem Spülanschluss (7) steht, bis zum distalen Ende des Hüllrohrs (5) erstreckt.

## Beschreibung

Die vorliegende Erfindung betrifft einen wiederverwendbaren Spülschaft für ein Endoskop mit einem Hauptteil und einem sich vom Hauptteil erstreckenden starren Endoskopschaft, der an seinem vom Hauptteil wegweisenden distalen Ende ein Deckglas aufweist.

Bisher bekannte Spülschäfte sind als Einmalprodukt ausgebildet, die nach Verwendung zu entsorgen sind.

Ausgehend hiervon ist es daher Aufgabe der Erfindung, einen verbesserten Spülschaft für ein Endoskop bereitzustellen.

Die Erfindung ist im Anspruch 1 definiert. Vorteilhafte Weiterbildungen sind in den abhängigen Ansprüchen angegeben.

Es wird ein wiederverwendbarer Spülschaft für ein Endoskop mit einem Hauptteil und einem sich vom Hauptteil erstreckenden starren Endoskopschaft, der an seinem vom Hauptteil wegweisenden distalen Ende ein Deckglas aufweist, bereitgestellt. Der Spülschaft umfasst einen proximalen Endabschnitt, der ein proximales Ende und ein distales Ende aufweist, ein mit dem distalen Ende des proximalen Endabschnitts verbundenes Hüllrohr, eine am proximalen Endabschnitt ausgebildete Fixiereinheit und einen Spülanschluss, der in Fluidverbindung mit dem Hüllrohr steht. Der Spülanschluss kann bevorzugt am proximalen Endabschnitt ausgebildet sein. Ferner umfasst der Spülschaft einen sich vom proximalen Ende des proximalen Endabschnitts durch den proximalen Endabschnitt und das Hüllrohr erstreckenden Endoskopkanal, so dass der Endoskopschaft mit seinem distalen Ende über das proximale Ende des proximalen Endabschnitts in den Endoskopkanal einführbar ist, bis für das Endoskop eine Arbeitsposition erreicht ist. Dazu umfasst das Hüllrohr an seinem vom proximalen Endabschnitt wegweisenden distalen Ende einen axialen Anschlag, der die maximale Eintrittstiefe des Endoskopschafts in den Endoskopkanal begrenzt, sowie mindestens zwei radiale Anschläge, die die radiale Position des Endoskopschafts im Hüllrohr vorgeben. Wenn der Endoskopschaft in den Endoskopkanal eingeführt und die Arbeitsposition erreicht ist, ist der Spülschaft mittels der Fixiereinheit so am Endoskop lösbar fixiert bzw. lösbar befestigt, dass das distale Ende des Endoskopschafts am axialen Anschlag anliegt und die radiale Position des Endoskopschafts im Hüllrohr durch die mindestens zwei radialen Anschläge so vorgegeben ist, dass sich ein Spülkanal innerhalb des Hüllrohrs, der in Fluidverbindung mit dem Spülanschluss steht, bis zum distalen Ende des Hüllrohrs erstreckt. Damit kann eine Flüssigkeit (bzw. Spülflüssigkeit), die über den Spülanschluss zugeführt wird, durch den Spülkanal und über das Deckglas, dass in der Arbeitsposition am distalen Ende des Hüllrohrs positioniert ist, strömen, um dieses zu reinigen. Dies kann insbesondere bei der bestimmungsgemäßen Verwendung des Endoskops durchgeführt werden.

Der proximale Endabschnitt, der Spülanschluss sowie das Hüllrohr können jeweils aus Edelstahl hergestellt sein. Dadurch ist eine Wiederverwendbarkeit des Spülschafts möglich.

Insbesondere kann das Hüllrohr mit dem proximalen Endabschnitt verklebt und/oder verschweißt sein. In gleicher Weise kann der Spülanschluss mit dem proximalen Endabschnitt verklebt und/oder verschweißt sein.

Die Fixiereinheit kann insbesondere so ausgebildet sein, dass in der Arbeitsposition der axiale Anschlag mit einer vorbestimmten Kraft gegen das distale Ende des Endoskopschafts drückt, so dass in axialer Richtung eine vorbestimmte Klemmwirkung vorliegt.

Der Spülanschluss kann an einer Mündungsstelle in den Endoskopkanal münden, wobei an einer Position, die näher am proximalen Ende des proximalen Endabschnitts als die Position der Mündungsstelle liegt, eine Ringnut mit einer eingesetzten Ringdichtung zum Abdichten des Endoskopkanals bei eingesetztem Endoskop vorgesehen sein kann.

Die Ringdichtung kann beispielsweise als O-Ringdichtung ausgebildet sein. Ferner kann die Ringdichtung zur Hemmung einer axialen Bewegung zwischen Endoskopschaft und Spülschaft dienen.

Aufgrund der von der Fixiereinheit bereitgestellten Klemmwirkung kann die Ringnut und die Ringdichtung so ausgelegt sein, dass eine relativ geringe Hemmwirkung vorliegt. So kann z.B. für einen Endoskopschaft mit einem Außendurchmesser von 4 mm im trocknen Zustand eine Kraft im Bereich von 3,5 N bis 5,8 N ausreichen, um den Endoskopschaft durch das distale Ende im Endoskopkanal und dabei an der Dichtung vorbei zu führen.

Durch diese geringe Hemmwirkung der Ringdichtung ist es ohne Probleme möglich, den Spülschaft mehrere 100 Mal auf einen Endoskopschaft aufzustecken und wieder abzuziehen. Wenn die Ringnut und die Dichtung so dimensioniert wären, dass größere Kräfte notwendig wären, um den Endoskopschaft einzuführen, würde dies relativ schnell zu einem Abscheren oder Beschädigen der Dichtung führen, so dass eine Wiederverwendbarkeit nicht gewährleistet werden könnte.

Die Fixiereinheit kann ferner einen Aufnahmeabschnitt aufweisen, in dem ein Teil des Endoskops (beispielsweise der Hauptteil) sitzt oder an dem ein Teil des Endoskops anliegt, wenn der Endoskopschaft in den Endoskopkanal eingeführt und die Arbeitsposition erreicht ist. Insbesondere kann der Aufnahmeabschnitt eine konkave Grundform aufweisen.

Die Fixiereinheit kann einstückig mit dem proximalen Endabschnitt ausgebildet sein.

Ferner können die mindestens zwei radialen Anschläge einstückig mit dem Hüllrohr ausgebildet sein. Insbesondere können die mindestens zwei radialen Anschläge zum Inneren des Hüllrohr vorstehen und durch eine Verdickung der Wandung des Hüllrohrs so ausgebildet sein, dass die Außenkontur des Hüllrohrs über die gesamte Länge (mit Ausnahme des Bereiches des axialen Anschlags) und somit auch im gesamten Bereich der radialen Anschläge konstant ist. Damit kann das Hüllrohr eine durchgehende glatte Außenfläche aufweisen, was zum Autoklavieren und daher zur Wiederverwendbarkeit des Spülschafts von Vorteil ist.

Die mindestens zwei radialen Anschläge können in Längsrichtung des Hüllrohrs gesehen abgerundet ausgebildet sein. Damit ist ein Einführen des Endoskopschafts und ein Herausziehen des Endoskopschafts ohne Probleme möglich, da durch die abgerundete Ausbildung in Längsrichtung ein Verkanten oder eine Beschädigung des distalen Endes des Endoskopschafts vermieden werden kann.

Ferner kann auch der axiale Anschlag einstückig mit dem Hüllrohr ausgebildet sein. Auch der axiale Anschlag kann durch eine Verdickung der Wandung des Hüllrohrs realisiert werden, die ins Innere des Hüllrohrs vorsteht. Somit kann auch im Bereich des axialen Anschlages eine glatte durchgehende Außenfläche des Hüllrohrs bereitgestellt werden.

Die zwei radialen Anschläge sowie der axiale Anschlag können in einer Ansicht auf das distale Ende des Hüllrohrs auf den Ecken eines gleichschenkligen Dreiecks sitzen.

Ferner ist es möglich, dass die zumindest zwei radialen Anschläge (bevorzugt genau zwei radiale Anschläge) so angeordnet sind, dass der eingeführte Endoskopschaft gegenüber dem Hüllrohr verkippt ist, wobei der Drehpunkt der Verkippung im Bereich des proximalen Endes des Endoskopkanals liegt. Im Bereich des Drehpunktes ist der Endoskopschaft bevorzugt zentriert angeordnet.

Es ist jedoch auch möglich, dass drei radiale Anschläge (bevorzugt genau drei radiale Anschläge) vorgesehen und so angeordnet sind, dass der eingeführte Endoskopschaft koaxial zum Hüllrohr angeordnet ist, so dass der Spülkanal einen ringförmigen Querschnitt aufweist.

Die zwei radialen Anschläge können in Umfangsrichtung insbesondere um 90° voneinander beabstandet sein. Der Abstand kann auch größer sein und beispielsweise 120°, 130° oder 140° betragen. Ferner ist es möglich, dass der Abstand geringer ist, beispielsweise 80°, 70°, 60° oder 45° beträgt.

Bei dem Spülschaft können drei radiale Anschläge vorgesehen sein, die in Umfangsrichtung bevorzugt äquidistant voneinander beabstandet sind.

Der Innendurchmesser des Hüllrohrs ist bevorzugt größer als der Außendurchmesser des Endoskopschafts. Insbesondere kann der Innendurchmesser des Hüllrohrs um 1% bis 10% (z.B. 2%, 3%, 4%, 5%, 6%, 7%, 8%, 9%, 9,5%, 9,6%, 9,7% oder 9,8%) größer sein als der Außendurchmesser des Endoskopschafts.

Die radialen Anschläge können beispielsweise im Bereich von 0,10 bis 0,30 mm gegenüber der Innenseite der Wandung des Hüllrohrs vorstehen. Damit kann ein Spülkanal bereitgestellt werden, der eine maximale radiale Ausdehnung im Bereich von 0,10 bis 0,30 mm aufweist.

Ferner können mehrere axiale Anschläge vorgesehen sein, die in Umfangsrichtung voneinander beabstandet sind. Insbesondere können die mehreren axialen Anschläge in Umfangsrichtung voneinander äquidistant beabstandet sein.

Das Hüllrohr kann als einwandiges Hüllrohr ausgebildet sein. Die Wandstärke der Wandung kann insbesondere mit Ausnahme der Bereiche, in denen die Anschläge gebildet sind, konstant sein.

Ferner kann das Hüllrohr als starres und/oder sich geradlinig erstreckendes Hüllrohr ausgebildet sein.

Das Hüllrohr kann hohlzylinderförmig ausgebildet sein. Das Hüllrohr kann einen runden, ovalen oder kreisförmigen Außen- und/oder Innen-Querschnitt aufweisen.

Das distale Ende des Hüllrohrs (insbesondere aufgrund des axialen Anschlags) ist insbesondere so ausgebildet, dass der Endoskopschaft mit seinem distalen Ende nicht über das distale Ende des Hüllrohrs in den Endoskopkanal einführbar ist. Somit kann z.B. ein nicht bestimmungsgemäßen Einführen des Endoskopschaftes in den Spülschaft sicher verhindert werden.

Das Hüllrohr kann an seinem distalen Ende eine distale Öffnung und an seinem vom distalen Ende wegweisenden proximalen Ende eine proximale Öffnung aufweisen.

Das Hüllrohr kann im Bereich vom proximalen Endabschnitt bis zu seinem distalen Ende bis auf die distale Öffnung frei von weiteren Öffnungen sein. Insbesondere kann das Hüllrohr bis auf seine distale und proximale Öffnung frei von weiteren Öffnungen sein.

Das Hüllrohr (bzw. die Wandung des Hüllrohrs) kann im Bereich vom proximalen Endabschnitt bis zu seinem distalen Ende eine durchgehend glatte Außenfläche aufweisen. Unter einer solchen durchgehend glatten Außenfläche wird insbesondere eine Außenfläche verstanden, die bis auf die distale und proximale Öffnung frei von Öffnungen ist, die sich in das Innere des Hüllrohrs erstrecken. Unter einer solchen durchgehend glatten Außenfläche wird ferner insbesondere eine Außenfläche verstanden, die keine Vertiefungen oder ähnliches und somit z.B. kein Kanten oder ähnliches aufweist.

Der Spülschaft kann insbesondere so ausgebildet sein, dass er (insbesondere als Ganzes) autoklavierbar ist.

Des Weiteren wird ein Endoskop zusammen mit dem wiederverwendbaren Spülschaft bereitgestellt.

Das Endoskop kann insbesondere als Sinuskop ausgebildet sein.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbeispiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine Schnittansicht einer Ausführungsform des erfindungsgemäßen Spülschafts 1 ;
- Fig. 2: eine Draufsicht auf den Spülschaft 1 gemäß Fig. 1;
- Fig. 3: eine Seitenansicht eines Endoskops 15;
- Fig. 4: eine Seitenansicht des Spülschafts 1 mit eingeführtem Endoskop 15 gemäß Fig. 3;
- Fig. 5: eine vergrößerte Schnittdarstellung des distalen Endes 25 des Hüllrohrs 5;
- Fig. 6: eine vergrößerte Schnittdarstellung des distalen Endes 25 des Hüllrohrs 5 mit eingeführtem Endoskopschaft 17;
- Fig. 7: eine perspektivische Darstellung des distalen Endes 25 des Hüllrohrs 5;
- Fig. 8: eine weitere perspektivische Darstellung des distalen Endes 25 des Hüllrohrs 5;
- Fig. 9: eine Ansicht vom proximalen Endabschnitt 2 auf das distale Ende 25 des Hüllrohrs 5;
- Fig. 10: eine Schnittansicht des Hüllrohrs 5 im Bereich der radialen Anschläge 27, 28;
- Fig. 11: eine Schnittansicht des distalen Endes des Hüllrohrs 5 einer weiteren Ausführungsform des Spülschafts 1 mit eingesetztem Endoskopschaft 17;
- Fig. 12: eine perspektivische Ansicht des distalen Endes 25 des Hüllrohrs 5 gemäß Fig. 11;
- Fig. 13: eine Schnittansicht des Hüllrohrs 5 gemäß Fig. 11 im Bereich der radialen Anschläge 27, 28 und 29;
- Fig. 14: eine Schnittansicht des distalen Endes des Hüllrohrs 5 einer weiteren Ausführungsform des Spülschaftes 1 mit eingesetztem Endoskopschaft 17;
- Fig. 15: eine perspektivische Ansicht des distalen Endes des Hüllrohrs 5 gemäß Fig. 14;
- Fig. 16: eine Schnittansicht des Hüllrohrs 5 gemäß Fig. 14 im Bereich der radialen Anschläge 27, 28;
- Fig. 17: eine Schnittansicht des distalen Endes des Hüllrohrs 5 in einer weiteren Ausführungsform des Spülschaftes 1 mit eingesetztem Endoskopschaft 17;
- Fig. 18: eine perspektivische Ansicht des distalen Endes 25 des Hüllrohrs 5 gemäß Fig. 17, und
- Fig. 19: eine Schnittansicht des Hüllrohrs 5 von Fig. 17 im Bereich der radialen Anschläge 27, 28 und 29.

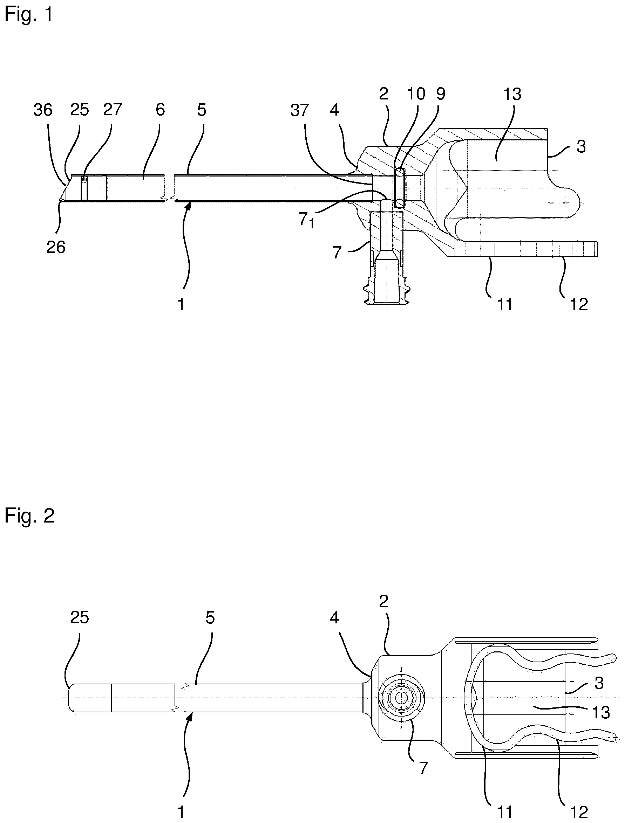

Bei der in den Fig. 1 und 2 gezeigten Ausführungsform des erfindungsgemäßen wiederverwendbaren Spülschafts 1 für ein Endoskop umfasst der Spülschaft 1 einen proximalen Endabschnitt 2 mit einem proximalen Ende 3 und einem distalen Ende 4. Am distalen Ende 4 ist ein Hüllrohr 5 ausgebildet, das z.B. hohlzylinderförmig mit einem kreisförmigen Querschnitt ist. Das Hüllrohr 5 weist eine distale Öffnung 36 und eine proximale Öffnung 37 auf und kann mit dem Endabschnitt 2 verklebt und/oder verschweißt sein.

Wie insbesondere Fig. 1 zu entnehmen ist, weist der Spülschaft 1 einen sich vom proximalen Ende 3 des Endabschnitts 2 durch den Endabschnitt 2 und das Hüllrohr 5 erstreckenden Endoskopkanal 6 auf.

Ferner ist am proximalen Endabschnitt 2 ein Spülanschluss 7 ausgebildet, der in Fluidverbindung mit dem Endoskopkanal 6 steht. Der Spülanschluss 7 kann mit dem Endabschnitt 2 verklebt und/oder verschweißt sein. Wie insbesondere in Fig. 1 ersichtlich ist, sitzt eine Dichtung 9 (beispielsweise eine O-Ring-Dichtung 9) in einer Ringnut 10, die näher am proximalen Ende 3 ausgebildet ist als die Mündungsstelle 7₁ des Spülanschlusses 7 in den Endoskopkanal 6.

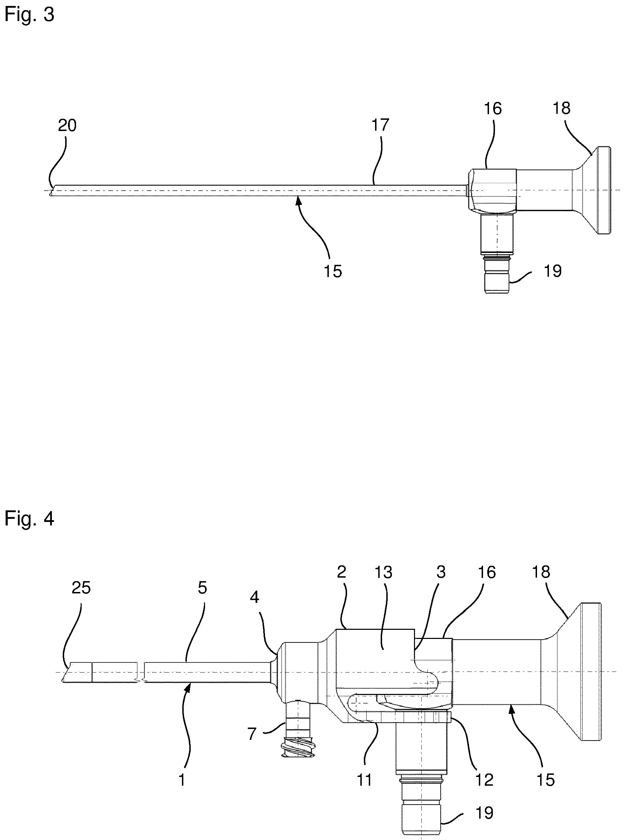

Der proximale Endabschnitt 2 umfasst ferner eine Fixiereinheit 11, mit der der Spülschaft 1 lösbar an einem Endoskop 15, wie es beispielsweise in Fig. 3 gezeigt ist, befestigbar ist, wie nachfolgend noch im Detail beschrieben ist. Die Fixiereinheit 11 umfasst hier eine elastische Klammer 12, wie z.B. in der Draufsicht gemäß Fig. 2 erkennbar ist, sowie einen Aufnahmeabschnitt 13. Die Klammer 12 ist hier einstückig mit dem restlichen proximalen Endabschnitt 2 ausgebildet. Der proximale Endabschnitt 2, das Hüllrohr 5 sowie der Spülanschluss 7 sind aus Edelstahl hergestellt, so dass der Spülschaft 1 insgesamt autoklavierbar ist.

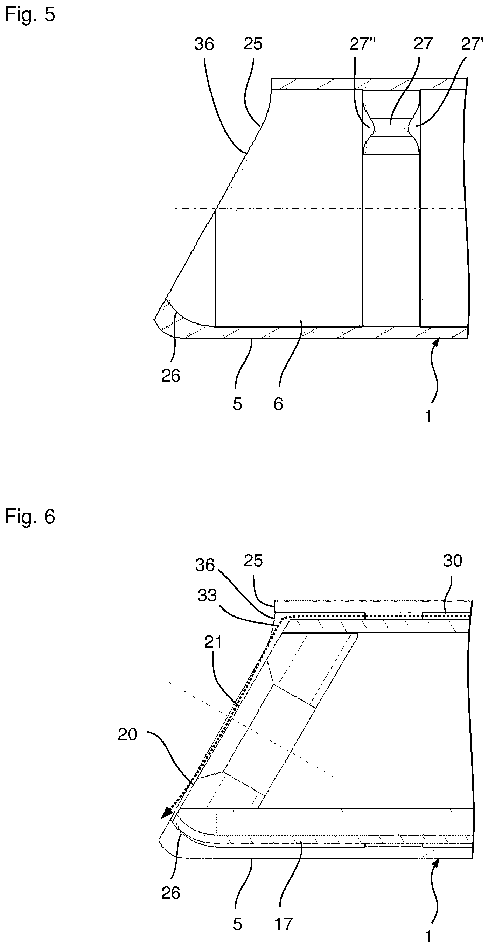

Das Endoskop 15, für das der Spülschaft 1 ausgelegt ist, weist einen Hauptteil 16, einen sich vom Hauptteil 16 erstreckenden starren Endoskopschaft 17, ein am Hauptteil 16 vorgesehenes Okular 18 sowie eine Lichtleiteranschluss 19 auf (wie in Fig. 3 dargestellt ist). Das Endoskop 15 weist ferner eine Beobachtungsoptik (nicht gezeigt) auf, die sich durch den Endoskopschaft 17 erstreckt, wobei das distale Ende 20 des Endoskopschafts 17 mit einem Deckglas 21 (Fig. 6) abgeschlossen ist. Somit kann ein Betrachter durch das Okular 18 den Bereich vor dem distalen Ende 20 in bekannter Art und Weise beobachten. Dazu kann insbesondere an den Lichtleiteranschluss 19 eine Lichtquelle angeschlossen sein, so dass über den Endoskopschaft 17 auch gleich die Beleuchtung des Bereiches vor dem distalen Ende 20 durchgeführt wird. Das hier gezeigte Endoskop 15 ist ein Sinuskop mit einer Blickrichtung von 30° bezogen auf die Längsachse des Endoskopschafts 17.

Der Spülschaft 1 ist so ausgebildet, dass der Innendurchmesser des Endoskopkanals 6 und insbesondere der Innendurchmesser des Hüllrohrs 5 größer ist als der Außendurchmesser des Endoskopschaftes 17. So kann der Innendurchmesser des Hüllrohrs z.B. 1 %-10% größer sein als der Außendurchmesser des Endoskopschaftes 17. Wenn der Außendurchmesser des Endoskopschaftes 17 z.B. 3,1 mm beträgt, kann der Innendurchmesser des Hüllrohrs 5 3,4 mm betragen. Wenn der Außendurchmesser des Endoskopschaftes 17 z.B. 4,0 mm beträgt, kann der Innendurchmesser des Hüllrohrs 5 4,2 mm betragen.

Der Spülschaft 1 kann über das distale Ende 20 des Endoskopschafts 17 auf den Endoskopschaft 17 geschoben werden bis der Hauptteil 16 im Aufnahmeabschnitt 13 sitzt bzw. an diesem anliegt und die Klammer 12 der Fixiereinheit 11 den Lichtleiteranschluss 19 federnd umgreift, wie in der Seitenansicht gemäß Fig. 4 schematisch dargestellt ist.

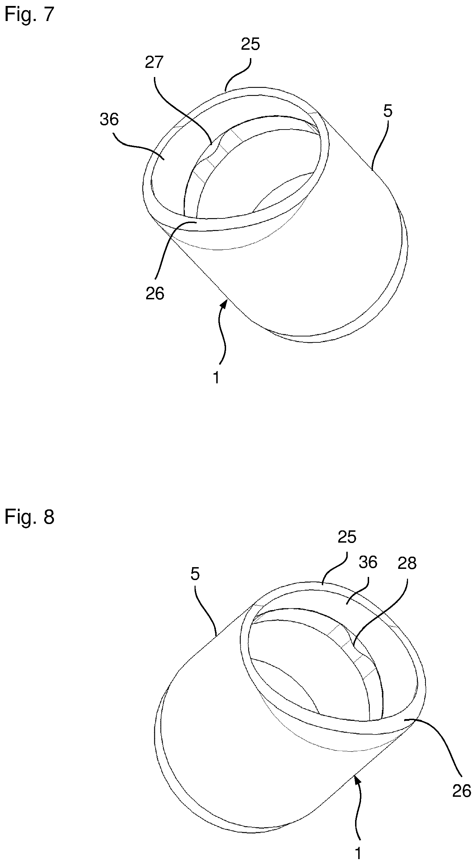

Wie z.B. in den Darstellungen gemäß Fig. 5 bis 10 erkennbar ist, ist der Spülschaft 1 an seinem distalen Ende 25 so ausgebildet, dass er einen distalen Anschlag 26 für das distale Ende 20 des Endoskopschafts 17 aufweist. Ferner weist die Wandung des Hüllrohrs 5 im Bereich des distalen Endes des Spülschaftes 1 zwei radiale Anschläge 27, 28 auf.

Die Abmessungen des Spülschafts 1 sind so gewählt, dass in der in Fig. 4 gezeigten Arbeitsstellung das distale Ende 20 des Endoskopschafts 17 am distalen Anschlag 26 anliegt.

Dabei wird aufgrund der Fixierung des Spülschafts 1 am Endoskop 15 mittels der Fixiereinheit 11 eine gewisse Kraft ausgeübt, die den distalen Anschlag 26 gegen das distale Ende 20 des Endoskopschafts 17 drückt. Somit ist die axiale Position des Endoskopschafts 17 in der Arbeitsstellung festgelegt.

Durch die beiden radialen Anschläge 27 und 28, an denen der Endoskopschaft 17 anliegt, wird auch die radiale Position des Endoskopschafts 17 vorgegeben. Diese radiale Position ist dabei so vorgegeben, dass der Endoskopschaft 17 und das Hüllrohr 5 nicht koaxial zueinander ausgerichtet sind, sondern zueinander verkippt. So drücken die radialen Anschläge 27, 28 den Endoskopschaft 17 zu der den radialen Anschlägen 27, 28 gegenüberliegenden Seite des Hüllrohrs 5, wohingegen im Bereich der Dichtung 9 der Endoskopschaft 17 zentriert im Endoskopkanal 6 positioniert ist. Somit liegt der Drehpunkt der Verkippung des Endoskopschafts 17 im Bereich der Dichtung 9 (bzw. im Bereich des proximalen Endes 3 des Endabschnitts 2 oder im Bereich des Aufnahmeabschnitts 13). Die Fixiereinheit 11 ist so ausgebildet, dass eine solche Verkippung möglich ist.

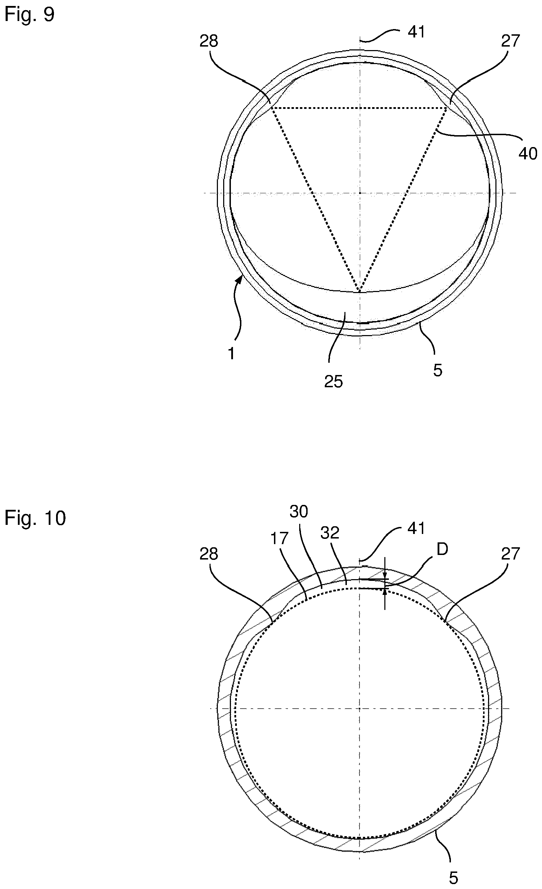

Aufgrund dieser Verkippung sowie des Innendurchmessers des Hüllrohrs 5, der größer ist als der Außendurchmesser des Endoskopschafts 17, ist zwischen dem Endoskopschaft 17 und der Wandung des Hüllrohrs 5 ein Spülkanal 30 ausgebildet, der von der Mündungsstelle 7₁ des Spülanschlusses 7 bis zum distalen Ende 25 des Hüllrohrs 5 verläuft. Im Bereich der Mündungsstelle 7₁ weist der Spülkanal 30 aufgrund der dort vorliegenden zentrierten Positionierung des Endoskopschaftes 17 im Hüllrohr 5 einen den Endoskopschaft 17 umgebenden ringförmigen Abschnitt auf. In Richtung zum distalen Ende 25 des Spülschaftes 1 hin wird aufgrund der Verkippung des Endoskopschafts 17 im Hüllrohr 5 der Spülkanal 30 immer mehr hauptsächlich durch einen Abschnitt 32 gebildet, der einen ringsegmentförmigen Querschnitt zwischen den beiden radialen Anschlägen 27 und 28 aufweist, wie in Fig. 10 dargestellt ist. Der Abschnitt 32 weist eine maximale radiale Ausdehnung D auf, die z.B. im Bereich von 0,10 bis 0,20 mm für Endoskope 15 mit einem Außendurchmesser des Endoskopschafts 17 von 4 mm liegt. Bei einem Spülschaft 1 für Endoskope 15 mit einem Außendurchmesser des Endoskopschafts 17 von 3,1 mm kann die maximale radiale Ausdehnung im Bereich von 0,20 bis 0,30 mm liegen. Dadurch wird erreicht, dass der Hauptteil der Spülflüssigkeit durch diesen Abschnitt 32 am distalen Ende 25 des Spülschaftes 1 (über die distale Öffnung 36) austritt. Damit wird über den Spülanschluss 7 zugeführte Spülflüssigkeit entlang des Spülkanals 30 bis zum distalen Ende 25 des Hüllrohrs 5 und dort sicher über das Deckglas 21 des Endoskopschafts 17 strömen, wie durch den Pfeil 33 in Fig. 6 angedeutet ist. In dieser Art und Weise kann ein Reinigen des Deckglases 21 mittels der Spülflüssigkeit z.B. während der bestimmungsgemäßen Nutzung des Endoskops 15 sichergestellt werden.

Die Dichtung 9 dient zur Abdichtung des Spülkanals 30 gegenüber dem proximalen Ende 3. Ferner dient die Dichtung 9 auch zur Hemmung einer axialen Bewegung zwischen Endoskopschaft 17 und Spülschaft 1. Da aufgrund der Fixiereinheit 11 in Verbindung mit dem distalen Anschlag 26 schon eine ausreichende Fixierung zwischen Spülschaft 1 und Endoskop 15 vorliegt, können die Ringnut 10 und die Dichtung 9 so ausgelegt werden, dass die Hemmwirkung der Dichtung 9 relativ gering ist. Für ein Endoskop 15 mit einem Außendurchmesser des Endoskopschafts 17 von 4 mm wird z.B. im trockenen Zustand eine Kraft im Bereich von 3,5 N bis 5,8 N benötigt, um den Endoskopschaft 17 durch das proximale Ende 3 in den Endoskopkanal 6 einzuführen und dabei an der Dichtung 9 vorbei zu führen. Dies bringt den Vorteil mit sich, dass es ohne Probleme möglich ist, den Spülschaft 1 100 bis 500 Mal auf ein Endoskop 15 aufzustecken und wieder abzuziehen und dabei dazwischen auch zu autoklavieren. Damit liegt eine Wiederverwendbarkeit des Spülschafts 1 vor.

Die beiden radialen Anschläge 27 und 28 sind hier nockenförmig ausgebildet. Insbesondere können die radialen Anschläge 27, 28 durch eine lokale Verdickung der Wandung des Hüllrohrs 5 gebildet sein, so dass die radialen Anschläge 27, 28 Teil der Wandung des Hüllrohrs 5 und somit einstückig mit dem Hüllrohr 5 ausgebildet sind. Ferner ist damit die Außenkontur des Hüllrohrs 5 im Querschnitt über die gesamte Länge des Hüllrohrs 5 gleich (mit Ausnahme des Bereiches mit dem axialen Anschlag 26). Damit ist die Außenseite des Hüllrohrs 5 eine glatte durchgehende Fläche, was z.B. für das Autoklavieren und somit auch für die Wiederverwendbarkeit vorteilhaft ist. Auch der distale Anschlag 26 kann einstückig mit dem Hüllrohr 5 ausgebildet sein. Auch hier ist eine lokale Verdickung oder eine entsprechende Formgebung möglich.

Wie der Ansicht auf das distale Ende 25 des Hüllrohrs 5 in Fig. 9 sowie der Schnittansicht in Fig. 10 zu entnehmen ist, sind die beiden radialen Anschläge 27, 28 (bzw. die beiden Nocken 27, 28) in Umfangsrichtung um 90° voneinander beabstandet. Der Abstand kann aber auch größer sein und beispielsweise 120°, 130° oder 140° betragen. Ferner ist es möglich, dass der Abstand geringer ist, und beispielsweise 80°, 70°, 60° oder 45° beträgt. Der axiale Anschlag 26 weist in Umfangsrichtung zum ersten und zweiten radialen Anschlag 27 und 28 jeweils betragsmäßig den gleichen Abstand auf. In dem hier gezeigten Ausführungsbeispiel beträgt er 135°. Somit sind die Anschläge 26 bis 28 in der Ansicht gemäß Fig. 9 an den Ecken eines gleichschenkligen Dreiecks 40 angeordnet, das lediglich zur Verdeutlichung gestrichelt in Fig. 9 eingezeichnet ist. Die beiden radialen Anschläge 27 und 28 sind spiegelsymmetrisch zu einem Durchmesser 41 angeordnet, wobei der axiale Anschlag 26 auf diesem Durchmesser 41 liegt.

Wie insbesondere der Darstellung in Fig. 5 zu entnehmen ist, ist der radiale Anschlag 27 so ausgebildet, dass er in Längsrichtung des Hüllrohrs 5 abgerundet und somit abgerundete Abschnitte 27' und 27" aufweist. Der radiale Anschlag 28 ist in gleicher Weise wie der radiale Anschlag 27 so ausgebildet, dass er in Längsrichtung des Hüllrohrs 5 abgerundet ist. Dadurch kann sichergestellt werden, dass beim Einführen des Endoskopschaftes 17 das distale Ende 20 des Endoskopschaftes 17 durch den Abschnitt 27' geführt und somit über den Nocken 27 geleitet wird. Der Abschnitt 27" dient beim Zurückziehen dazu, ein Verkanten oder Verkeilen zu verhindern. Somit kann der Endoskopschaft 17 leicht in das Hüllrohr 5 eingeführt und aus dem Hüllrohr 5 wieder herausgezogen werden.

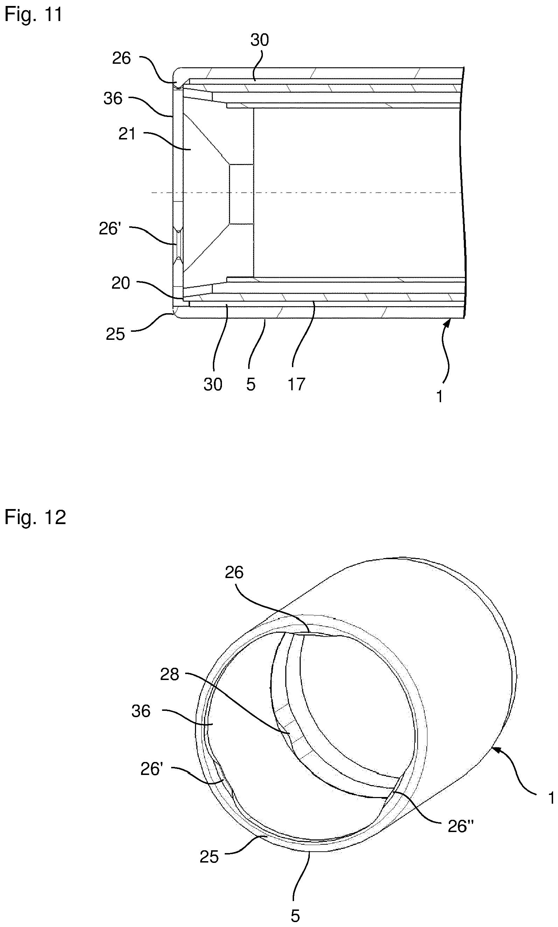

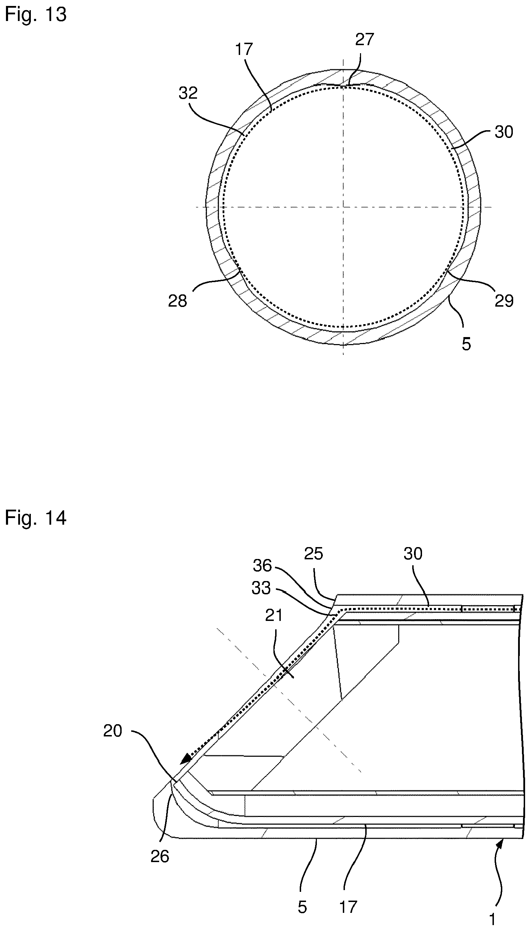

In den Fig. 11 bis 13 ist eine Abwandlung des Spülschafts 1 gezeigt. Bei dieser Abwandlung ist der Spülschaft 1 für ein Endoskop 15 mit einer Blickrichtung von 0° ausgebildet, wobei in diesem Fall drei radiale Anschläge 27, 28, 29 vorgesehen sind, so dass der Endoskopschaft 17 in der Arbeitsstellung koaxial im Hüllrohr 5 positioniert ist. Die drei radialen Anschläge 27 bis 29 sind in Umfangsrichtung jeweils um 120° voneinander beabstandet und jeweils so ausgebildet, dass sie in Längsrichtung des Hüllrohrs 5 abgerundet sind. In gleicher Weise sind drei distale Anschläge 26, 26' und 26" vorgesehen, die ebenfalls in Umfangsrichtung um 120° voneinander beabstandet sind. Damit liegt ein Spülkanal 30 mit im Wesentlichen ringförmigem Querschnitt vor, der lediglich im Abschnitt 32 der radialen Anschläge 27-29 im Querschnitt durch die radialen Anschläge 27-29 unterbrochen ist, wie in Fig. 13 schematisch dargestellt ist. Man kann daher auch sagen, dass der Spülkanal 30 einen ringförmigen Querschnitt aufweist.

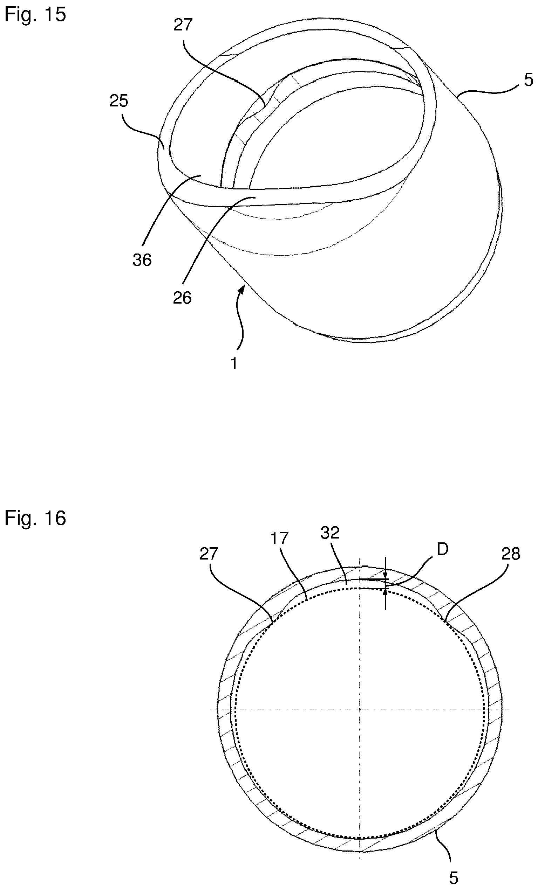

In Fig. 14 bis 16 ist eine Variante des Spülschafts 1 für ein Endoskop 15 mit einer Blickrichtung von 45° dargestellt. Wie den Darstellungen entnommen werden kann, sind zwei radiale Anschläge 27 und 28 vorgesehen, so dass der Endoskopschaft 17 nicht koaxial sondern verkippt zum Hüllrohr 5 verläuft und ein Spülkanal 30 wie bei der Ausführungsform gemäß Fig. 1-10 vorliegt. Ferner ist ein radialer Anschlag 26 vorgesehen.

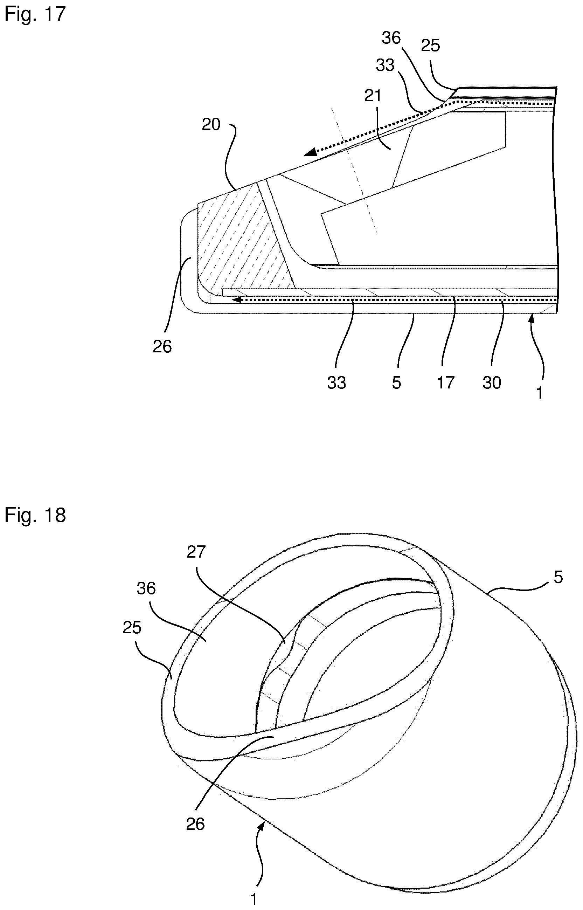

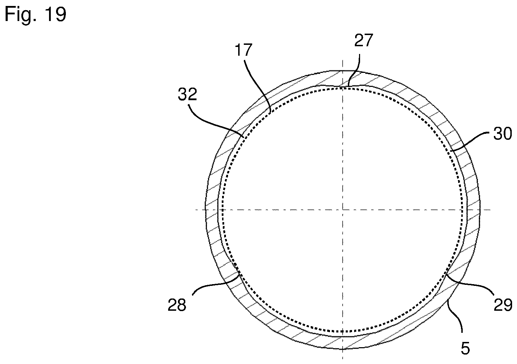

In Fig. 17 bis 19 ist ein Ausführungsbeispiel des Spülschafts 1 gezeigt, das für ein Endoskop 15 mit einer Blickrichtung von 70° ausgebildet ist. In diesem Fall sind drei radiale Anschläge 27 bis 29 vorgesehen, wie in Fig. 19 dargestellt ist. Ferner ist ein distaler Anschlag 26 vorgesehen, so dass die Lage des Endoskopschafts 17 in der Arbeitsstellung vorgegeben ist. Durch die drei radialen Anschläge 27-29 liegt wieder ein Spülkanal 30 mit ringförmigem Querschnitt vor.

## Patentansprüche

1. Wiederverwendbarer Spülschaft (1) für ein Endoskop (15) mit einem Hauptteil (16) und einem sich vom Hauptteil (16) erstreckenden starren Endoskopschaft (17), der an seinem vom Hauptteil (16) wegweisenden distalen Ende (20) ein Deckglas (21) aufweist, wobei der Spülschaft (1)
einen proximalen Endabschnitt (2), der ein proximales Ende (3) und ein distales Ende (4) umfasst,
ein mit dem distalen Ende (4) des proximalen Endabschnitts (2) verbundenes Hüllrohr (5),
eine am proximalen Endabschnitt (2) ausgebildete Fixiereinheit (11) und
einen Spülanschluss (7), der in Fluidverbindung mit dem Hüllrohr (5) steht, aufweist,
wobei der Spülschaft (1) einen sich vom proximalen Ende (3) des proximalen Endabschnitts (2) durch den proximalen Endabschnitt (2) und das Hüllrohr (5) erstreckenden Endoskopkanal (6) aufweist, so dass der Endoskopschaft (17) mit seinem distalen Ende (20) über das proximale Ende (3) des proximalen Endabschnitts (2) in den Endoskopkanal (6) einführbar ist, bis für das Endoskop (15) eine Arbeitsposition erreicht ist,
wobei das Hüllrohr (5) an seinem vom proximalen Endabschnitt (2) wegweisenden distalen Ende (25) einen axialen Anschlag (26), der die maximale Einführtiefe des Endoskopschafts (17) in den Endoskopkanal (6) begrenzt, sowie mindestens zwei radiale Anschläge (27, 28, 29), die die radiale Position des Endoskopschafts (17) im Hüllrohr (5) vorgeben, aufweist,
wobei der Spülschaft (1), wenn der Endoskopschaft (17) in den Endoskopkanal (6) eingeführt und die Arbeitsposition erreicht ist, mittels der Fixiereinheit (11) so am Endoskop (15) fixiert ist, dass das distale Ende (20) des Endoskopschafts (17) am axialen Anschlag (26) anliegt und die radiale Position des Endoskopschafts (17) im Hüllrohr (5) durch die mindestens zwei radialen Anschläge (27-29) so vorgegeben ist, dass sich ein Spülkanal (30) innerhalb des Hüllrohrs (5), der in Fluidverbindung mit dem Spülanschluss (7) steht, bis zum distalen Ende (25) des Hüllrohrs (5) erstreckt.

2. Spülschaft nach Anspruch 1, bei dem
der proximale Endabschnitt (2), der Spülanschluss (7) sowie das Hüllrohr (5) jeweils aus Edelstahl hergestellt sind.

3. Spülschaft nach Anspruch 1 oder 2,
bei dem der Spülanschluss (7) an einer Mündungsstelle (7₁) in den Endoskopkanal (6) mündet,
wobei an einer Position, die näher am proximalen Ende (3) des proximalen Endabschnitts (2) liegt, eine Ringnut (10) mit einer eingesetzten Ringdichtung (9) zum Abdichten des Endoskopkanals (6) bei eingesetztem Endoskopschaft (17) vorgesehen ist.

4. Spülschaft nach einem der obigen Ansprüche, bei dem
die Fixiereinheit (11) einstückig mit dem proximalen Endabschnitt (2) ausgebildet ist.

5. Spülschaft nach einem der obigen Ansprüche, bei dem
die mindestens zwei radialen Anschläge (27, 28) einstückig mit dem Hüllrohr (5) ausgebildet sind.

6. Spülschaft nach einem der obigen Ansprüche, bei dem
die mindestens zwei radialen Anschläge (27, 28) zum Inneren des Hüllrohrs (5) vorstehen und durch eine Verdickung der Wandung des Hüllrohrs (5) so ausgebildet sind, dass das Hüllrohr (5) eine durchgehend glatte Außenfläche aufweist,
wobei bevorzugt die mindestens zwei radialen Anschläge (27, 28) in Längsrichtung des Hüllrohrs abgerundet ausgebildet sind.

7. Spülschaft nach einem der obigen Ansprüche, wobei
die Fixiereinheit (11) so ausgebildet ist, dass in der Arbeitsposition der axiale Anschlag (26) mit einer vorbestimmten Kraft gegen das distale Ende (20) des Endoskopschafts (15) drückt, so dass in axialer Richtung eine vorbestimmte Klemmwirkung vorliegt.

8. Spülschaft nach einem der obigen Ansprüche, wobei
der axiale Anschlag (26) einstückig mit dem Hüllrohr (5) ausgebildet ist.

9. Spülschaft nach einem der obigen Ansprüche, bei dem
die zwei radialen Anschläge (27, 28) und der axiale Anschlag (26) in einer Ansicht auf das distale Ende (25) des Hüllrohrs (5) auf den Ecken eines gleichschenkligen Dreiecks sitzen.

10. Spülschaft nach einem der obigen Ansprüche, bei dem
die mindestens zwei radialen Anschläge (27, 28) so angeordnet sind, dass der eingeführte Endoskopschaft (17) gegenüber dem Hüllrohr (5) verkippt ist, wobei der Drehpunkt der Verkippung im Bereich des proximalen Endes (2) des proximalen Endabschnitts (2) liegt,
oder bei dem drei radiale Anschläge (27-29) vorgesehen und so angeordnet sind, dass der eingeführte Endoskopschaft (17) koaxial im Hüllrohr (5) positioniert ist.

11. Spülschaft nach einem der Ansprüche 1 bis 8, bei dem
drei radiale Anschläge vorgesehen sind, die in Umfangsrichtung äquidistant voneinander beabstandet sind.

12. Spülschaft nach einem der obigen Ansprüche, bei dem
mehrere axiale Anschläge (26, 26`, 26") vorgesehen sind, die in Umfangsrichtung voneinander beabstandet sind.

13. Spülschaft nach einem der obigen Ansprüche, bei dem
das Hüllrohr (5) als einwandiges das Hüllrohr (5) ausgebildet ist.

14. Spülschaft nach einem der obigen Ansprüche, bei dem
das Hüllrohr (5) an seinem distalen Ende (25) eine distale Öffnung (36) aufweist und bei dem das Hüllrohr (5) im Bereich vom proximalen Endabschnitt (2) bis zu seinem distalen Ende (25) bis auf die distale Öffnung (36) frei von weiteren Öffnung ist.

15. Endoskop (15) mit einem wiederverwendbarer Spülschaft (1) nach einem der obigen Ansprüche,
wobei das Endoskop (15) ein Hauptteil (16) und einen sich vom Hauptteil (16) erstreckenden starren Endoskopschaft (17), der an seinem vom Hauptteil (16) wegweisenden distalen Ende (20) ein Deckglas (21) aufweist, umfasst.
